# EUROPÄISCHE PATENTSCHRIFT

(11) **EP 0 941 070 B1**
(45) Veröffentlichungstag und Bekanntmachung des Hinweises auf die Patenterteilung: **26.03.2003**
(21) Anmeldenummer: 97950197.0
(22) Anmeldetag: 17.11.1997
(51) Int. Cl.: A61K 9/20

(54) **LUTSCHTABLETTE ZUR MODIFIZIERTEN FREISETZUNG VON WIRKSTOFFEN IM GASTROINTESTINALTRAKT**
LOZENGE FOR THE MODIFIED RELEASING OF ACTIVE SUBSTANCES IN THE GASTROINTESTINAL TRACT
PASTILLE POUR LA LIBERATION MODIFIEE DE PRINCIPES ACTIFS DANS LE TRACTUS GASTRO-INTESTINAL

(30) Priorität: 23.11.1996 DE 19648576
(43) Veröffentlichungstag der Anmeldung: 15.09.1999
(73) Patentinhaber: LTS Lohmann Therapie-Systeme AG, 56626 Andernach (DE)
(72) Erfinder: CREMER, Karsten, D-53119 Bonn (DE)
(74) Vertreter: Flaccus, Rolf-Dieter, Dr.
(86) Internationale Anmeldenummer: EP9706395
(87) Internationale Veröffentlichungsnummer: WO98023262

(56) Entgegenhaltungen:
- EP-A- 0 212 745
- EP-A- 0 601 508
- WO-A-91/19486
- FR-A- 2 565 107
- PATENT ABSTRACTS OF JAPAN vol. 013, no. 069 (C-569), 16.Februar 1989 & JP 63 258809 A (TOWA YAKUHIN KK), 26.Oktober 1988, & DATABASE WPI Section Ch, Week 8849 Derwent Publications Ltd., London, GB; Class A12, AN 88-348774 & JP 63 258 809 A (TOWA YAKUHIN KK) , 26.Oktober 1988 & CHEMICAL ABSTRACTS, vol. 111, no. 6, 7.August 1989 Columbus, Ohio, US; abstract no. 45317, FUKUI Y. ET AL: "Pharmaceutical granule coating for the control of bitter taste without altering bioavailability"

## Beschreibung

Die Erfindung betrifft eine pharmazeutische Lutschtablette zur modifizierten Freisetzung von Wirkstoffen im Gastrointestinaltrakt mit wirkstoffhaltigen Partikeln, welche eine erste, die Freisetzung kontrollierende Umhüllung und eine weitere, äußere Umhüllung mit speichelresistenten Eigenschaften aufweisen.

Peroral applizierbare Darreichungsformen mit modifizierter Wirkstofffreisetzung können verschiedenen therapeutischen Zielsetzungen dienen. Die häufigsten Ziele sind der Schutz der Magenschleimhaut vor einem schädigenden Wirkstoff oder der des Wirkstoffes vor dem sauren Milieu im Magen einerseits, was durch magensaftresistente Umhüllungen bewirkt wird; außerdem die Kontrolle der Freisetzungsgeschwindigkeit über eine längere Zeit, was durch verschiedene retardierende Maßnahmen erreicht werden kann und zu Plasmaspiegeln mit relativ geringen Schwankungen, einer dadurch besseren Verträglichkeit und einer längeren Wirksamkeit der Applikation führt.

Eine Formulierung mit modifizierter Wirkstofffreisetzung läßt sich nicht bei allen pharmakologisch geeigneten Wirksubstanzen gleichermaßen leicht entwickeln. Sie erfordert in der Regel eine Reihe von Zusatzstoffen, mit deren Hilfe sich die erwünschten Effekte erzielen lassen. Diese Zusatzstoffe erhöhen dementsprechend deutlich die Masse der Darreichungsform. Bei Darreichungsformen mit prolongierter Wirkstofffreisetzung ist außerdem die zu applizierende Dosis gegenüber einer einfachen Darreichungsform erhöht. Bei verschiedenen Wirkstoffen ließen sich deshalb aufgrund ihrer Dosis mit den bisher bekannten Möglichkeiten keine Darreichungsformen mit prolongierter Wirkstofffreisetzung entwickeln, da sie wegen der resultierenden Dimensionen nicht in Form einer Kapsel oder Tablette geschluckt werden könnten. So müssen beispielsweise verschiedene Antibiotika nach wie vor drei- bis viermal täglich oral appliziert werden, obwohl man weiß, daß bei einer mehr als zweimal täglichen Anwendung die Zuverlässigkeit der Einnahme durch Patienten gering ist. Bei Antibiotika liegt die nichtretardierte Einmaldosis häufig bereits bei 500 bis 1000 mg, was zusammen mit den zur Arzneiformung notwendigen Hilfsstoffen zu nur noch schwer zu schluckenden Kapseln oder Tabletten führt. Ein auf der Basis konventioneller Techniken hergestelltes Retardpräparat mit mehr als 1000 mg Wirkstoff und einem nochmals erhöhten Anteil an Hilfsstoffen läßt sich praktisch nicht mehr schlucken.

Wünschenswert ist daher die Bereitstellung einer leicht und angenehm einzunehmenden Darreichungsform mit modifizierter Wirkstofffreisetzung, welche auch für Wirkstoffe geeignet ist, die in hohen Einmaldosen eingenommen werden müssen.

So offenbaren die Offenlegungsschriften EP 0 601 508, WO 91/194 86, FR 2 565 107 und JP 63 258 809 unverpreßte Wirkstoffzubereitungen in Form von zweischichtig umhüllten Partikeln, deren äußere Umhüllungsschicht speichelresistent aber magensaftlöslich ist. Die innere Umhüllungsschicht der Partikel gemäß EP 0 601 508 und JP 63 258 809 läßt eine retardierte Wirkstofffreisetzung zu oder ist, wie bei den in WO 91/194 86 und FR 2 565 107 beschriebenen Partikeln, magensaftresistent, jedoch im Dünndarm löslich. Die bekannten zweischichtig umhüllten Wirkstoffzubereitungen werden als Granulate oder in Wasser dispergiert verabreicht.

Erfindungsgemäß soll das Problem der Einnahme hoher Dosen zunächst dadurch gelöst werden, daß eine zu einer Lutschtablette verpreßte, multipartikuläre Wirkstoffzubereitung verabreicht wird, deren Partikel einzeln zur Erzielung einer modifizierten Freigabe umhüllt vorliegen. Die umhüllten Partikel werden beim Lutschen der Tablette im Mund in fein verteilter Form freigesetzt und können mit dem Speichel leicht geschluckt werden.

Die Applikationsform der Lutschtablette ist seit langem bekannt und wird häufig angewendet, um Wirkstoffe an die erkrankte Mund- und Rachenschleimhaut abzugeben. Auch für systemisch wirksame Substanzen kann die Lutschtablette geeignet sein, wenn die Substanzen einen annehmbaren Geschmack besitzen und ohne Verzögerung zur Resorption gebracht werden sollen. Für die Lösung des Problems wird die Anwendungsform der Lutschtablette eingesetzt, weil sie mehr Wirk- und Hilfsstoffmasse ohne Verlust ihrer Applizierbarkeit aufzunehmen vermag als eine zum Schlucken bestimmte Tablette. Lutschtabletten mit einer Masse von mehr als 4 g befinden sich im Handel und werden von Patienten offenbar problemlos akzeptiert.

Prinzipiell erlaubt der Stand der Technik das Verpressen von umhüllten Partikeln mit modifizierter Wirkstofffreisetzung, obwohl der erfindungsgemäße Verwendungszweck mit seinen Vorteilen - die Applikation als Lutschtablette mit Eignung auch für hohe Wirkstoffdosen - weder als Lösung des weiter oben dargestellten Problems erkannt noch beschrieben wurde.

So lehren beispielsweise die Patentschriften EP 153 104 und EP 355 247 das Verpressen von wirkstoffhaltigen, retardierend umhüllten Partikeln zu Tabletten, ein Verfahren, das auch in weiteren Quellen beschrieben wird. Darüber hinaus offenbart die Patentschrift US 5 464 632 eine zum Schlucken oder zum Zerfall im Mund vorgesehene, schnellzerfallende Tablette, die den Wirkstoff optional in Form von überzogenen Partikeln zur modifizierten Freisetzung enthält. Allerdings ist bei dieser Tablette im Falle des Zerfalls im Mund zu erwarten, daß Anteile des Wirkstoffes durch die Umhüllung der Partikel bereits in der Mundhöhle freigesetzt werden und dabei einen negativen Geschmackseindruck hinterlassen.

Analog löst auch die Applikationsform der Lutschtablette, wie sie in der vorliegenden Erfindung gefordert wird, einerseits zwar das Problem der Applizierbarkeit größerer Wirk- und Hilfsstoffmengen, schafft jedoch andererseits neue Probleme. Zum einen werden bei magensaftresistent umhüllten Partikeln die Umhüllungen dem pH-neutralen Speichel ausgesetzt, wodurch sie vorzeitig zerfallen und den gewünschten Schutzeffekt im Magen nicht mehr erzielen können. Zum anderen werden bei Partikeln, die mit retardierenden Umhüllungen versehen sind, Anteile des Wirkstoffes durch Diffusion bereits im Mundraum freigesetzt, was insbesondere bei unangenehm schmeckenden Substanzen nicht akzeptabel ist.

Daher besteht ein Bedarf an einer weiter verbesserten Darreichungsform mit modifizierter Wirkstofffreisetzung zur Verabreichung hoher Wirkstoffdosen, welche die aufgeführten Nachteile nicht aufweisen. Diese der Erfindung zugrundeliegende Aufgabe wird durch eine pharmazeutische Darreichungsform nach einem der beiden Hauptansprüche gelöst: Neben der speziellen Ausgestaltung als Lutschtablette überwindet die erfindungsgemäße Tablette die Nachteile des Standes der Technik dadurch, daß ihr Wirkstoff in Form von zweifach bzw. zweischichtig umhüllten Partikeln vorliegt, wobei eine erste, innere Umhüllung der modifizierten Wirkstofffreisetzung dient, während eine weitere, äußere Umhüllung speichelresistent ist, sich im sauren Milieu des Magens jedoch auflöst und hierdurch sicherstellt, daß während des Lutschens der Tablette nicht bereits Wirkstoff im Mundraum freigesetzt wird. Die erste, innere Umhüllung der Partikel kann nach einem der beiden Hauptansprüche die Wirkstofffreisetzung entweder dadurch modifizieren, daß die Freisetzung erst nach der Magenpassage der Partikel einsetzt, oder sie kann als im Magen- und Darmsaft unlösliche Membran ausgebildet sein, durch die der Wirkstoff jedoch langsam nach außen diffundieren und retardiert freigesetzt werden kann.

Erfindungsgemäß werden die wirkstoffhaltigen Partikel zusammen mit geeigneten Hilfsstoffen zu Lutschtabletten gepreßt, die bedingt durch ihre Zusammensetzung im Mund langsam erodieren und dabei die Partikel in den Speichel abgeben. Der Speichel mit den umhüllten Partikeln wird daraufhin geschluckt. Selbstversuche ergaben, daß Partikel bis zu etwa 100 - 200 µm Durchmesser beim Lutschen als nur mäßig störend empfunden werden.

Filmbildende Polymere, die zur Erzeugung einer speichelresistenten Umhüllung eingesetzt werden können, sind dem Fachmann bekannt. Häufig wird für diesen Zweck ein Copolymerisat auf der Basis von Dimethylaminoethylmethacrylat und neutralen Methacrylsäureestern mit der Markenbezeichnung Eudragit E (Fa. Röhm) eingesetzt. Der basische Charakter des Dimethylaminoethylmethacrylats sorgt für eine Löslichkeit im sauren Milieu, wie z. B. im Magensaft, während die Löslichkeit im relativ neutralen Speichel eher gering ist. Bei geeigneter Filmdicke läßt sich daher mit diesem Copolymerisat eine Umhüllung erzeugen, die dem Zerfall im Speichel für eine längere Zeit widersteht, die sich aber im Magen rasch auflöst. Alternativ lassen sich alle anderen filmbildenden Polymere für diesen Zweck verwenden, welche eine im Magensaft deutlich bessere Löslichkeit als im Speichel aufweisen.

Freisetzungsretardierende Filmüberzüge sind in der Technologie der festen oralen Darreichungsformen weit verbreitet. Die hierzu eingesetzten Polymere weisen typischerweise eine geringe Löslichkeit in wäßrigen Medien sowohl bei sauren als auch bei neutralen bis basischen pH-Werten auf. Bei ausreichender Dicke und mechanischer Festigkeit des Filmüberzugs löst sich dieser also weder im Speichel noch im Magen- oder Darmsaft auf. Andererseits darf die Dicke nicht zu groß sein, denn der Filmüberzug muß die Diffusion von Wasser in das Wirkstoffreservoir erlauben, wo der Wirkstoff kontinuierlich gelöst wird und in gelöster Form durch die Umhüllung nach außen diffundiert. Eine Vielzahl von Polymeren wurde für diesen Zweck eingesetzt; als Beispiele seinen genannt: Polymere aus der Gruppe der Celluloseester, wie etwa Celluloseacetat, Vertreter der Gruppe der Celluloseether, wie z. B. Ethylcellulose, bestimmte Poly(meth)acrylsäurederivate, z. B. Eudragit® RL bzw. RS (Fa. Röhm), bestimmte Polyvinylderivate wie Polyvinylacetat.

Die typischerweise zur Herstellung von magensaftresistenten, jedoch dünndarmsaftlöslichen Filmumhüllungen verwendeten Polymere weisen eine äußerst geringe Löslichkeit im sauren Milieu bei deutlich besserer Löslichkeit im neutralen pH-Bereich auf. Diese Eigenschaften zeigen sich vor allem bei solchen Polymeren, die saure Gruppen enthalten, die im Magensaft undissoziiert vorliegen. Als Beispiele seinen genannt:

Halbester zweiwertiger Säuren wie Bernstein- oder Phthalsäure mit Celluloseethern, Celluloseestern, Polyvinylderivaten, beispielsweise Polyvinylacetatsuccinat oder Polyvinylacetatphthalat, mit Carboxymethylcellulosen oder Polyacrylsäuren bzw. Polymethacrylsäuren, wie Eudragit® L bzw. S (Fa. Röhm) enthalten sind.

Elastizität und Festigkeit der Filmumhüllungen der Partikel sind eine Voraussetzung für die Funktionstüchtigkeit der Arzneiform, denn die Umhüllungen dürfen durch die starke mechanische Beanspruchung beim Verpressen bzw. Tablettieren nicht versehrt werden; zumindest soll die überwiegende Mehrzahl der in der Lutschtablette enthaltenen Partikel zwei intakte Umhüllungsschichten im Sinne der Erfindung besitzen. Dem Fachmann ist bekannt, daß die Elastizität, Flexibilität und Festigkeit der Polymerfilme abhängig ist vom Polymertyp, Molekulargewicht, Substitutionsgrad der eingesetzten Filmbildner, aber auch von der Art und Menge der eingesetzten Zusatzstoffe. Insbesondere Weichmacher oder zu anderen Zwecken eingesetzte, jedoch weichmachende Zusatzstoffe haben auf die mechanischen Filmeigenschaften einen erheblichen Einfluß. Für die meisten im Sinne der Erfindung einsetzbaren Polymere kennt der Stand der Technik geeignete Weichmacher zur Erzeugung bestimmter mechanischer Eigenschaften; der Fachmann wird durch geeignete Versuche in der Lage, die für den Zweck der erfindungsgemäßen Tablettierung von umhüllten Partikeln optimale Weichmachermenge zu ermitteln, die von der für andere Zwecke empfohlenen Menge durchaus abweichen kann. Erfindungsgemäß können pharmazeutische Lutschtabletten, wie sie hierin zuvor beschrieben sind, durch ein Verfahren hergestellt werden, bei dem zunächst eine multipartikuläre Zubereitung in einem ersten Verfahrensschritt durch ein Agglomerisationsverfahren aus einer Mischung aus Wirkstoff und geeigneten Hilfsstoffen hergestellt wird. In einem zweiten Verfahrensschritt werden die Partikel in einem Sprühauftragsverfahren zunächst mit der die Wirkstofffreisetzung retardierenden oder magensaftresistenten Filmschicht und anschließend mit einer speichelresistenten, jedoch magensaftlöslichen Filmschicht umhüllt. In einem dritten Verfahrensschritt werden die umhüllten Partikel zusammen mit weiteren Hilfsstoffen auf einer Tablettenmaschine zu Lutschtabletten verpreßt.

Zur Verdeutlichung möglicher Ausführungen der Erfindung mögen zwei Beispiele dienen, welche jedoch nur illustrierenden Charakter besitzen.

### Beispiel 1: Lutschtabletten mit magensaftresistent umhüllten Ibuprofenpartikeln mit 600 mg Ibuprofen

Zunächst werden durch Granulieren, Extrudieren und Sphäronisieren Ibuprofen-Mikropellets hergestellt. Hierzu werden 700 g Ibuprofen, 180 g mikrokristalline Cellulose und 120 g Lactose-monohydrat in einem Pulvermischer gemischt und anschließend in einem geeigneten Mischkneter unter Zugabe von n g Wasser zu einer Masse mit knetbarer Konsistenz angeteigt. Die Masse wird mit einem Extruder, z. B. einem Einschnecken-Extruder vom Typ E 40/10 D (Fa. Gabler) durch eine sondergefertigte Lochscheibe mit einem Lochdurchmesser von 300 µm extrudiert, geschnitten und möglichst im On-line-Verfahren, z. B. in einem Sphäronisierer vom Typ R 250 (Fa. Gabler) gerundet. Die Mikropellets werden anschließend in einem Trockner bis zur Restfeuchte von ca. 2-3% getrocknet, was in einem geeigneten Wirbelschichtgerät, vorzugsweise jedoch bereits in dem für das Umhüllen der Pellets vorgesehenen Gerät, z. B. dem Wirbelschicht-Granulator/Trockner/Coater vom Typ Uni-Glatt (Fa. Glatt) durchgeführt werden kann.

Zur Erzeugung des ersten, magensaftresistenten Überzuges werden die Pellets in der Wirbelschicht über eine Zweistoffdüse bei ca. 1-2 bar in Top-Spray-Anordnung und einer Sprühgeschwindigkeit von 10 ml/min mit einer Dispersion aus 95 g Eudragit® L 30 D-55, 45 g Eudragit® NE 30 D (beides Fa. Röhm), 8 g Triethylcitrat, 12 g Polyethylemglykol 6000, 20 g Talkum und 90 g Wasser bis zu einer Trockengewichtszunahme von 12% besprüht. Eine geeignete Sprühtemperatur ist 38-43°C, wobei eine Nachtrocknung bei 30-35°C erfolgen sollte.

Die so hergestellten, magensaftresistent umhüllten Partikel werden anschließend erfindungsgemäß mit einem zweiten, speichelresistenten Überzug versehen. Alternativ kann auch durch einen Wechsel des Sprühmediums im vorangegangenen Prozeß übergangslos die Herstellung der Speichelresistenz erfolgen. Eine geeignete Sprühlösung hierfür setzt sich aus 240 g Eudragit® E 12,5, 18 g Polyethylenglykol 6000, 12 g mikrokristalliner Cellulose, 12 g Magnesiumstearat und 220 g Aceton zusammen. Sie kann in der oben beschriebenen Apparatur bei gleichem Druck und gleicher Sprühgeschwindigkeit, vorzugsweise jedoch mit einer auf ca. 30°C herabgesetzten Temperatur aufgesprüht werden. Der Sprühvorgang wird bei einer Gewichtszunahme der Pellets von 11% beendet.

Nach dem Nachtrocknen können die nunmehr mit zwei Überzügen versehenen Partikel zu Lutschtabletten verpreßt werden. Dazu werden 1066 g Pellets - diese enthalten 600 g Ibuprofen - in einem Pulvermischer mit 260 g Sorbit (notwendig ist eine direkttablettierbare Qualität), 2 g kolloidalem Siliciumdioxid, 28 g Stearinsäure und 11 g Magnesiumstearat gemischt und auf einer Tablettenpresse zu 1367 mg schweren Tabletten, z. B. mit einem Durchmesser von 18 mm gepreßt.

### Beispiel 2: Lutschtabletten mit freisetzungsretardierend umhüllten Ibuprofenpartikeln mit 600 mg Ibuprofen

Die Herstellung geschieht analog Beispiel 1 mit der Ausnahme, daß zur Erzeugung des die Freisetzung modifizierenden Überzugs eine Dispersion aus 110 g Eudragit RS 30 D, 25 g Talkum, 28 g Triethylcitrat und 15 g Polyethylenglykol 6000 verwendet wird.

Diese Ausführungsbeispiele verdeutlichen das Prinzip der Erfindung; je nach Priorisierung der Produkteigenschaften, z. B. geringe Partikelgrößen zur sensorischen Verbesserung der Lutsch- oder Zerfallseigenschaften der Tablette oder geringere Hilfsstoffanteile zur Herabsetzung der Produktionskosten, lassen sie sich deutlich in Richtung eines der Zielparameter optimieren.

## Patentansprüche

1. Aus pulver- oder granulatförmiger Preßmasse gepreßte pharmazeutische Lutschtablette, **dadurch gekennzeichnet, daß** die Preßmasse neben üblichen, zur Herstellung von Lutschtabletten geeigneten Hilfsstoffen eine Wirkstoffzubereitung in Form von mindestens zweischichtig umhüllten Partikeln enthält, wobei eine äußere Umhüllungsschicht speichelresistent, jedoch magensaftlöslich, und eine innere Umhüllungsschicht in wäßrigen Medien weitgehend zerfallsresistent ist, eine retardierte Wirkstofffreisetzung durch Diffusion jedoch zuläßt.

2. Aus pulver- oder granulatförmiger Preßmasse gepreßte pharmazeutische Lutschtablette, **dadurch gekennzeichnet, daß** die Preßmasse neben üblichen, zur Herstellung von Lutschtabletten geeigneten Hilfsstoffen eine Wirkstoffzubereitung in Form von mindestens zweischichtig umhüllten Partikeln enthält, wobei eine äußere Umhüllungsschicht speichelresistent, jedoch magensaftlöslich, und eine innere Umhüllungsschicht magensaftresistent, jedoch dünndarmlöslich ist.

3. Pharmazeutische Lutschtablette nach Anspruch 1 oder 2, **dadurch gekennzeichnet, daß** die speichelresistente, jedoch magensaftlösliche Umhüllungsschicht als Filmbildner ein oder mehrere Polymere aus der Gruppe der Dimethylaminoethylmethacrylate und der Methacrylsäureester enthält.

4. Pharmazeutische Lutschtablette nach Anspruch 1, **dadurch gekennzeichnet, daß** die die Wirkstofffreisetzung retardierende Umhüllungsschicht als Filmbildner ein oder mehrere Polymere aus der Gruppe der Celluloseether, Celluloseester, Polyacrylsäurederivate, Polymethacrylsäurederivate und Polyvinylderivate enthält.

5. Pharmazeutische Lutschtablette nach Anspruch 2, **dadurch gekennzeichnet, daß** die magensaftresistente Umhüllungsschicht als Filmbildner ein oder mehrere Polymere aus der Gruppe der Celluloseether-, Celluloseester-, Polyvinylacetatphthalate oder -succinate, der Carboxymethylethylcellulosen, der Polyacrylsäurederivate und der Polymethacrylsäurederivate enthält.

6. Pharmazeutische Lutschtablette nach einem der vorangegangenen Ansprüche, **dadurch gekennzeichnet, daß** die Filmumhüllungen neben filmbildenden Polymeren einen Gehalt an solchen pharmazeutisch akzeptablen Hilfsstoffen aufweisen, welche in der Lage sind, die Flexibilität der Filmumhüllungen zu erhöhen.

7. Verfahren zur Herstellung von pharmazeutischen Lutschtablette nach einem der vorangegangenen Ansprüche, **dadurch gekennzeichnet, daß** in einem ersten Verfahrensschritt durch ein Agglomerationsverfahren aus einer Mischung aus Wirkstoff und geeigneten Hilfsstoffen eine multipartikuläre Zubereitung hergestellt wird, deren Partikel in einem zweiten Verfahrensschritt in einem Sprühauftragsverfahren zunächst mit einer die Wirkstofffreisetzung retardierenden oder magensaftresistenten Filmschicht, dann mit einer speichelresistenten, doch magensaftlöslichen Filmschicht umhüllt und in einem dritten Verfahrensschritt zusammen mit weiteren Hilfsstoffen auf einer Tablettenmaschine zu Lutschtabletten verpreßt werden.

## Claims

1. Pharmaceutical lozenge pressed from pulverulent or granular press material, **characterized in that** the press material contains an active-compound preparation in the form of at least two-layer coated particles in addition to customary auxiliaries suitable for the production of lozenges, an outer coating layer being saliva-resistant, but soluble in gastric juice, and an inner coating layer being largely disintegration-resistant in aqueous media, but allowing a delayed release of active compound by diffusion.

2. Pharmaceutical lozenge pressed from pulverulent or granular press material, **characterized in that** the press material contains an active-compound preparation in the form of at least two-layer coated particles in addition to customary auxiliaries suitable for the production of lozenges, an outer coating layer being saliva-resistant, but soluble in gastric juice, and an inner coating layer being gastric juice-resistant, but soluble in the small intestine.

3. Pharmaceutical lozenge according to Claim 1 or 2, **characterized in that** the saliva-resistant, but gastric juice-soluble coating layer contains as film-forming agent one or more polymers from the group consisting of the dimethylaminoethyl methacrylates and the methacrylic acid esters.

4. Pharmaceutical lozenge according to Claim 1, **characterized in that** the coating layer delaying the release of active compound contains as film-forming agent one or more polymers from the group consisting of the cellulose ethers, cellulose esters, polyacrylic acid derivatives, polymethacrylic acid derivatives and polyvinyl derivatives.

5. Pharmaceutical lozenge according to Claim 2, **characterized in that** the gastric juice-resistant coating layer contains as film-forming agent one or more polymers from the group consisting of the cellulose ether, cellulose ester or polyvinyl acetate phthalates or succinates, the carboxymethylcelluloses, the polyacrylic acid derivatives and the polymethacrylic acid derivatives.

6. Pharmaceutical lozenge according to one of the preceding claims, **characterized in that** the film coatings, in addition to film-forming polymers, contain those pharmaceutically acceptable auxiliaries which are able to increase the flexibility of the film coatings.

7. Process for the production of pharmaceutical lozenges according to one of the preceding claims, **characterized in that** in a first process step, by means of an agglomeration process, a multiparticulate preparation is prepared from a mixture of active compound and suitable auxiliaries, whose particles are coated in a second process step in a spray application process first with an active-compound-delaying or enteric film coating, then with a saliva-resistant, but gastric juice-soluble film coating, and compressed together with further auxiliaries in a tablet machine in a third process step to give lozenges.

## Revendications

1. Pastille pharmaceutique comprimée à partir d'une matière moulée en poudre ou en granulés, **caractérisée en ce que** la matière moulée, outre les adjuvants habituels appropriés à la fabrication de pastilles, contient une préparation de principes actifs sous forme de particules enrobées d'au moins deux couches, une couche d'enrobage extérieure étant résistante à la salive mais soluble dans le suc gastrique et une couche d'enrobage intérieure étant résistante dans une large mesure à la décomposition en milieux acqueux, mais permettant cependant une libération prolongée par diffusion des principes actifs.

2. Pastille pharmaceutique comprimée à partir d'une matière moulée en poudre ou en granulés, **caractérisée en ce que** la matière moulée, outre les adjuvants habituels appropriés à la fabrication de pastilles, contient une préparation de principes actifs sous forme de particules enrobées d'au moins deux couches, une couche d'enrobage extérieure étant résistante à la salive mais soluble dans le suc gastrique et une couche d'enrobage intérieure étant résistante au suc gastrique mais soluble dans l'intestin grêle.

3. Pastille pharmaceutique selon la revendication 1 ou 2, **caractérisée en ce que** la couche d'enrobage résistante à la salive mais soluble dans le suc gastrique contient comme agent filmogène un ou plusieurs polymères du groupe des diméthylaminoéthylméthacrylates et des esters de l'acide méthacrylique.

4. Pastille pharmaceutique selon la revendication 1, **caractérisée en ce que** la couche d'enrobage prolongeant la libération des principes actifs contient comme agent filmogène un ou plusieurs polymères du groupe des éthers de cellulose, des esters de cellulose, des dérivés de l'acide polyacrylique, des dérivés de l'acide polyméthacrylique et des dérivés du polyvinyle.

5. Pastille pharmaceutique selon la revendication 2, **caractérisée en ce que** la couche d'enrobage résistante au suc gastrique contient comme agent filmogène un ou plusieurs polymères du groupe des phtalates ou succinates d'éthers et d'esters de cellulose, des phtalates ou succinates de polyacétate de vinyle, des carboxyméthyléthylcelluloses, des dérivés de l'acide polyacrylique et des dérivés de l'acide polyméthacrylique.

6. Pastille pharmaceutique selon l'une des revendications précédentes, **caractérisée en ce que** les enrobages pelliculaires, outre les polymères filmogènes, présentent une teneur en adjuvants acceptables au plan pharmaceutique capables d'augmenter la flexibilité des enrobages pelliculaires.

7. Procédé de fabrication de pastilles pharmaceutiues selon l'une des revendications précédentes, **caractérisé en ce que** dans une première étape une préparation multiparticulaire est fabriquée par agglomération à partir d'un mélange de principes actifs et d'adjuvants appropriés, dont les particules sont d'abord enrobées dans une seconde étape d'un procédé d'application par pulvérisation d'une couche pelliculaire prolongeant la libération des principes actifs ou résistante au suc gastrique, puis d'une couche pelliculaire résistante à la salive mais soluble dans le suc gastrique pour être comprimées en pastilles avec d'autres adjuvants sur une pastilleuse dans une troisième étape.
